# EUROPEAN PATENT SPECIFICATION

(11) **EP 0 728 491 B1**
(45) Date of publication and mention of the grant of the patent: **15.11.2000**
(21) Application number: 95120169.8
(22) Date of filing: 20.12.1995
(51) Int. Cl.: A61M 1/36, A61M 1/02, B01J 47/00

(54) **Method of producing a blood transfusion device**
Herstellungsverfahren für eine Bluttransfusionsvorrichtung
Méthode de production d'un dispositif de transfusion

(30) Priority: 22.02.1995 JP 5812495; 22.02.1995 JP 5812595; 22.02.1995 JP 5812695; 13.03.1995 JP 7937995
(43) Date of publication of application: 28.08.1996
(73) Proprietor: KAWASUMI LABORATORIES, INC., Tokyo (JP)
(72) Inventor: Inaba, Shoichi, Hukuoka-shi, Hukuoka-ken (JP); Shirahama, Noriaki, Kawasumi Lab., Inc., Mie-cho, Ohno-gun, Ohita-ken (JP); Takayanagi, Kenji, Kawasumi Lab., Inc., Mie-cho, Ohno-gun, Ohita-ken (JP); Fukumori, Yoshihiro, Kawasumi Lab., Inc., Mie-cho, Ohno-gun, Ohita-ken (JP); Yuasa, Takeshi, Kawasumi Lab., Inc., Mie-cho, Ohno-gun, Ohita-ken (JP)
(74) Representative: Heusler, Wolfgang, Dipl.-Ing.

(56) References cited:
- EP-A- 0 161 471
- WO-A-89/04687
- GB-A- 966 447
- GB-A- 982 107
- GB-A- 2 213 056
- US-A- 2 682 268
- US-A- 3 865 726
- US-A- 4 314 025
- US-A- 4 943 287
- DORFNER: 'Ion Exchangers', 1991, WALTER DE GRUYTER, NEW YORK * page 1412 *

## Description

### BACKGROUND OF THE INVENTION

This invention relates to a method of producing a device for transfusing a preserved blood preparation to be infused to a patient, said device comprising an ion concentration adjusting room.

### Prior art

In a medical institution, when a blood preparation is transfused to an immature baby, a renal insufficiency patient or a patient at heart operation, it cannot be sometimes transfused to the patient since a potassium ion concentration in plasma is increased if the blood preparation is preserved for a long term. Thus, washed erythrocytes have heretofore been used. However, this preparation cannot be preserved and is necessary to make reservation for use so that it cannot be sometimes obtained when intended to be used.

In recent years, there has been found a risk of generating GVHD caused by active leukocytes in the blood preparation after blood transfusion. As measures therefor, it can be considered to generally carry out irradiation to the blood preparation. However, in the irradiated blood preparation lymphocytes die out in leukocytes so that the GVHD can be prevented but the inactivated leukocytes are themselves admitted as antigens after blood transfusion so that the same kinds of antigens are formed whereby alloimmunization cannot be prevented. Also, the potassium ion concentration of the irradiated blood preparation increases during preservation so that irradiation must be carried out immediately before use whereby clinical correspondence is difficult in many cases.

In Japanese Patent Publication (Kokoku) Sho. 55-22101 (22101/1980), there is disclosed that an adsorption portion comprising a strongly acidic cation exchange resin is provided in the course of a blood transfusion circuit of a blood transfusion set to adsorb a potassium ion in the blood. However, an amount of the adsorbent to be packed is large whereby the adsorption portion becomes large-sized or it is not adapted to rapid blood transfusion so that it is not sufficiently satisfactory in constitution or characteristics.

Also, when a large amount of a blood preparation is transfused, a sodium ion is excessively loaded so that a dropsical swelling is sometimes caused whereby a continuous relaxation blood filtration is sometimes carried out to remove the sodium ion in the blood by using a blood filtration filter. However, the blood filtration is a treatment after a symptom is revealed. Thus, it has been desired to decrease an amount of the sodium ion to be loaded before effecting blood transfusion by maintaining the concentration of the sodium ion in the blood preparation to a low level.

On the other hand, in a rapid and mass blood transfusion accompanied with flooding, an exchange blood transfusion, use of a continuous blood components separation device or plasma exchange for hemolytic diseases of the newborn, citric acid is transfused accompanied with blood transfusion (autotransfusion) so that acute toxic symptoms are sometimes revealed in the case of mass blood transfusion. This is because a citric acid ion forms a complex with a calcium ion (Ca²⁺) in blood by a chelate reaction and decrease in the calcium ion (Ca²⁺) concentration causes the symptoms which is a main cause. In a slight symptom, numbness of the limbs or a chill is present but proceeding the symptoms, nausea or vomiting is sometimes accompanied by. These acute toxic symptoms due to citric acid can be improved by supplementing calcium so that when mass blood transfusion is carried out, calcium is often transfused depending on necessity while in view of the patient's condition. Particularly in a patient lowered in liver function or a light-weighted patient, these toxic symptoms may likely appear. Thus, it has been desired to remove excess citric acid (free citric acid, a citric acid ion).

GB-A-982 107 discloses a method of decreasing the citrate ion concentration of citrated blood, comprising placing a mixture of anion and cation exchange resins in a tube for contacting the blood, and passing several volumes of physiological saline solution through the column to render it isotonic to blood. The ends are protected and the unit is heat sterilized.

GB-A-2213056 discloses a method of manufacturing agents for removing human immunodeficiency virus and its related compounds contained in body fluid, in which a solid substance with a weakly acidic or weakly alkaline surface is used, and a method of evaluating the ability to remove HIV antigen and related compounds by circulation. The evaluation method comprises the steps of charging an ion concentration adjusting room containing ion exchange material with a priming solution harmless to a human body and carrying out a high pressure vapor sterilization (autoclaving) procedure. After sterilization by autoclaving, the saline solution in the sterilized column is replaced by a culture liquid, which is then circulated through the column, and after that the virus antigen concentration in the culture liquid is measured.

### SUMMARY OF THE INVENTION

A general object of the present invention is, before effecting transfusion of a preserved blood preparation to a patient, to decrease ion concentrations such as potassium ion, sodium ion, etc. contained in said preserved blood preparation or to remove excessive citric acid. A blood transfusion device to be produced according to the present invention comprises an introduction tube to introduce the preserved blood preparation, an injection needle to transfuse the preserved blood preparation to a patient and a cramp for adjusting a flow rate of the preserved blood preparation which is provided in the course of said induction tube, and an ion concentration adjusting room is provided in the course of the introduction tube of said preserved blood preparation and an ion concentration adjusting member which can adjust a specific ion concentration in blood is charged or packed in said ion concentration adjusting room.

Another object of the present invention is to make ion concentrations of the preserved blood preparation passed through the ion concentration adjusting room uniform. Thus a blood transfusion device shall be produced in which a blood storing portion to temporarily store the preserved blood preparation passed through said ion concentration adjusting room is provided at the downstream side to the above ion concentration adjusting room of the induction tube.

Other object of the present invention is to realize long term storage of the device by sterilely and easily washing and removing dissolved materials dissolved out from the ion concentration adjusting member. Thus, a blood transfusion device shall be provided in which a priming solution harmless to human body is filled up inside of the ion concentration adjusting room.

Still other object of the present invention is to adjust the ion concentrations of the preserved blood preparation and simultaneously to remove leukocytes so that hyperkalemia after transfusion is prevented and also side effects such as GVHD, etc. due to removal of leukocytes are prevented whereby safety of blood transfusion is improved. Thus, a blood transfusion device shall be provided in which a leukocyte removal filter for removing leukocytes in the preserved blood preparation is provided as well as the above ion concentration adjusting member.

These objects are achieved by the method according to Claim 1.

### BRIEF DESCRIPTION OF THE DRAWING

FIG. 1 is a schematic view showing a first Example of the device to be produced,
FIG. 2 is a schematic view showing a second Example,
FIG. 3 is a schematic view showing a third Example,
FIG. 4 is a schematic view showing a fourth Example,
FIG. 5 is a sectional schematic view along with the B-B line of FIG. 4,
FIG. 6 is a sectional schematic view showing another Example of a blood treating room of the above fourth Example,
FIG. 7 is a schematic view showing a fifth Example,
FIG. 8 is a sectional schematic view along with the C-C line of FIG. 7,
FIG. 9 is a schematic view showing a sixth Example,
FIG. 10 is a schematic view showing a used state of the above sixth Example, and
FIG. 11 is a schematic view showing a seventh Example of the device to be produced.

### DETAILED DESCRIPTION OF THE INVENTION

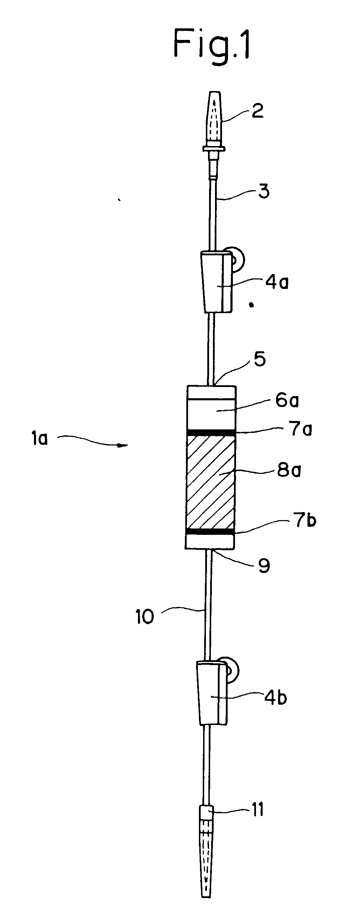

FIG. 1 is one example of a blood transfusion device for adjusting an ion concentration of a preserved blood preparation. This blood transfusion device la comprises an ion concentration adjusting room 6a, a connection tube 3 for introduction of the preserved blood preparation and a blood transfusion tube 10.

The ion concentration adjusting room 6a has an inlet 5 and an outlet 9 of the preserved blood preparation, and the connection tube 3 is connected with the inlet 5 and the blood transfusion tube 10 is connected with the outlet 9. At the tip portion of the connection tube 3, a connecting needle 2 for introducing the preserved blood is provided, and in the course of said tube 3, a roll cramp 4a is provided. Also, at the end portion of the blood transfusion tube 10, an injection needle 11 is provided, and in the course of said tube 10, a roll cramp 4b is provided. The connection tube 3 and the blood transfusion tube 10 are made of a flexible synthetic resin such as a vinyl chloride resin, etc.

In the aforesaid ion concentration adjusting room 6a, an ion concentration adjusting member 8a is provided and at both of the top and bottom end of the ion concentration adjusting member 8a, supporting filters 7a and 7b are provided, respectively.

For the ion concentration adjusting member 8a, a cation exchange resin or an anion exchange resin is used in the present invention.

As the cation exchange resin, those having an exchange ability of 1 to 5 mEq/ml and an average particle size of 0.2 to 1.5 mm, preferably an exchange ability of 1.5 to 3.5 mEq/ml and an average particle size of 0.4 to 1.0 mm are used. There may be used, for example, polystyrene sodium sulfonate, polystyrene calcium sulfonate, polystyrene sulfonic acid, sodium methacrylate, etc.

As the anion exchange resin, those having an exchange ability of 0.5 to 4 mEq/ml and an average particle size of 0.2 to 1.5 mm, preferably an exchange ability of 0.8 to 2.5 mEq/ml and an average particle size of 0.4 to 1.0 mm are used. There may be used, for example, those in which the terminal end is a Cl⁻ (chlorine ion) type one such as trimethyl ammonium chloride, etc., or a OH⁻ type one such as dimethylethanol amine, etc.

The ion concentration adjusting room 6a is constituted by a synthetic resin such as a vinyl chloride resin, etc., in a cylindrical or bag shape, and the top end portion to which the connection tube 3 is connected and the bottom end portion to which the blood tube 10 is connected are both closed. The aforesaid supporting filters 7a and 7b are provided to fix the ion concentration adjusting member 8a and to filtrate foreign substances.

When the preserved blood preparation is to be transfused to a patient according to the blood transfusion device 1a as shown in FIG. 1, a blood bag (not shown) in which the preserved blood preparation is contained therein is hanging down with a predetermined height, a connecting needle 2 is inserted into a blood transfusion inlet of the blood bag and roll cramps 4a and 4b which close the connecting tube 3 and a liquor transferring tube 10 are open. According to this operation, the preserved blood preparation in the blood bag is flown out due to difference of the heads to the connection tube 3, the ion concentration adjusting room 6a, the blood transfusion tube 10 and the injection needle 11 in this order. After confirming flow out from the injection needle 11, the roll cramp 4b of the blood transfusion tube 10 is once closed and the injection needle 11 is pricked to a blood vessel of a patient, thereafter the roll cramp 4b is gradually opened and a blood flow is adjusted to effect blood transfusion.

### Example 1

By using the blood transfusion device la shown in FIG. 1, a potassium ion concentration adjusting test in the preserved blood was carried out. As a preserved blood, 200 ml of blood preserved in a blood bag (a blood collecting volume of 200 ml) made of a soft polyvinyl chloride containing a CDP solution was introduced into an ion concentration adjusting room 6a, by controlling cramps 4a and 4b, through a connection tube 3 with a head of 70 cm. A flow down speed of the blood was 20 ml/min and the blood was collected in a receiving vessel. Inside of the ion concentration adjusting room 6a was packed 5 ml of polystyrene sodium sulfonate having an average particle size of 0.4 mm and an exchange ability of 1.8 mEq/ml (potassium ion) which is a cation exchange resin.

### Example 2

In the same manner as in Example 1, 200 ml of the preserved blood was introduced into an ion concentration adjusting room 6a, by controlling cramps 4a and 4b, through a connection tube 3 with a head of 70 cm. A flow down speed of the blood was 50 ml/min and the blood was collected in a receiving vessel. Inside of the ion concentration adjusting room 6a was packed 8 ml of polystyrene sodium sulfonate having an average particle size of 0.6 mm and an exchange ability of 1.8 mEq/ml (potassium ion) as a cation exchange resin. Potassium ion concentrations in the plasma before and after passing the ion concentration adjusting room 6a were measured by using an ion chromatograph device 4500i manufactured by DIONEX Co. The results of Example 1 and Example 2 are shown in Table 1.

**Table 1**

| Sample No. | Example 1 | | | | Example 2 | | | |
|---|---|---|---|---|---|---|---|---|
| | 1 | 2 | 3 | 4 | 5 | 6 | 7 | 8 |
| K⁺ concentration (mEq/L) Before passing ion concentration adjusting room | 9.5 | 16.7 | 27.5 | 38.6 | 8.8 | 15.8 | 30.4 | 38.4 |
| After passing ion concentration adjusting room | 2.7 | 5.2 | 7.1 | 9.5 | 2.4 | 5.1 | 8.1 | 9.3 |
| Removed ratio (%) | 71.6 | 68.8 | 74.2 | 75.4 | 72.7 | 67.7 | 73.4 | 75.8 |

In Example 1 and Example 2, examples of adjusting the potassium ion concentration are mentioned, but by setting the kind, size and volume of the ion exchange resin to a desired ion concentration, other ion concentrations in the blood such as a sodium ion and a calcium ion can be adjusted.

### Example 3

As a preserved blood, 100 ml, 200 ml, 300 ml or 400 ml of bloods preserved in a blood bag (a blood collecting volume of 200 ml to 400 ml) made of a soft polyvinyl chloride was introduced into an ion concentration adjusting room 6a, by controlling cramps 4a and 4b, through a connection tube 3 with a head of 70 cm. A flow down speed of the blood was made 20 ml/min and the blood was collected in a saucer. Inside of the ion concentration adjusting room 6a was packed 25 ml of polystyrene trimethyl ammonium chloride having an average particle size of 0.5 mm and an exchange ability of 1.5 mEq/ml as an anion exchange resin.

### Example 4

Inside of the ion concentration adjusting room 6a was packed 15 ml of polystyrene trimethyl ammonium chloride as in Example 3, and whole bloods (100 ml, 200 ml, 300 ml and 400 ml) preserved in a CPD solution were treated in the same manner as in Example 3.

### Example 5

Inside of the ion concentration adjusting room 6a was packed 15 ml of polystyrene trimethyl ammonium chloride as in Example 3, and conc. erythrocytes (100 ml, 200 ml and 300 ml) preserved in a MAP solution were treated in the same manner as in Example 3.

### Example 6

Inside of the ion concentration adjusting room 6a was packed 15 ml of polystyrene trimethyl ammonium chloride as in Example 3, and conc. erythrocytes (100 ml, 200 ml and 300 ml) preserved in a MAP solution were introduced into the ion concentration adjusting room 6a through a connection tube 3 in the same manner as in Example 3 and passed with a flow down speed of 100 ml/min to collect in a blood receiving vessel.

The results of Examples 3, 4, 5 and 6 are shown in Table 2.

**Table 2**

| | Example 3 | | | | | Example 4 | | | | |
|---|---|---|---|---|---|---|---|---|---|---|
| Citric acid content (W/V%) | Before filtration | After filtration (filtrated amount ml) | | | | Before filtration | After filtration (filtrated amount ml) | | | |
| | | 100 | 200 | 300 | 400 | | 100 | 200 | 300 | 400 |
| | 0.73 | 0.025 | 0.037 | 0.056 | 0.080 | 0.71 | 0.019 | 0.027 | 0.048 | 0.077 |
| Removed ratio (%) | - | 96.6 | 94.9 | 92.3 | 89.0 | - | 97.3 | 96.2 | 93.2 | 89.2 |

| | Example 5 | | | | | Example 6 | | | | |
|---|---|---|---|---|---|---|---|---|---|---|
| Citric acid content (W/V%) | Before filtration | After filtration (filtrated amount ml) | | | - | Before filtration | After filtration (filtrated amount ml) | | | - |
| | | 100 | 200 | 300 | | | 100 | 200 | 300 | |
| | 0.29 | 0.005 | 0.006 | 0.006 | - | 0.28 | 0.006 | 0.021 | 0.023 | - |
| Removed ratio (%) | - | 98.3 | 97.9 | 97.9 | - | - | 97.9 | 92.5 | 91.8 | - |

As clearly seen from Table 2, by using the anion exchange resin as an ion concentration adjusting material, an amount of the anticoagulant (citric acid) which enters in a body at component selective blood collection or blood transfusion can be markedly reduced whereby appearance of citric acid toxic symptom can be suppressed and blood transfusion can be safely carried out. Also, removal of citric acid can be carried out that 90 % or more thereof can be removed in a preparation (leukocytes, conc. erythrocytes, MAP-conc. erythrocytes, etc.) containing erythrocytes as two unit treatment. Further, for rapid and massblood transfusion, citric acid can be sufficiently removed sothat blood transfusion at flooding can be safely carried out.

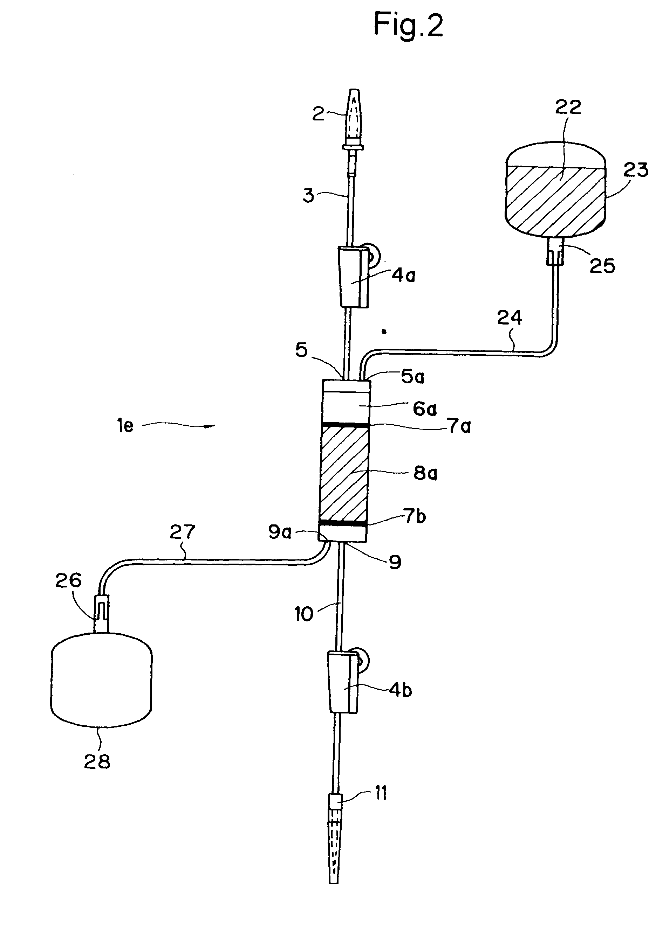

The blood transfusion device le shown in FIG. 2 is an example in which a priming treatment device is provided to the blood transfusion device shown in FIG. 1 whereby the priming treatment can be carried out. That is, in the ion concentration adjusting room 6a, an inlet 5a and an outlet 9a of a priming solution are provided and inside the ion concentration adjusting room 6a, a priming solution such as a physiological saline aqueous solution, a Ringer's solution, etc. is previously filled up.

At the inlet 5a of the ion concentration adjusting room 6a, a priming vessel 23 in which the above priming solution 22 is contained is connectqd through a connection tube 24, and at the connection port of the priming vessel 23 and the connection tube 24, a communication piece 25 having the constitution as mentioned above is equipped. Also, at the outlet 9a, a waste solution vessel 28 is connected through a connection tube 27, and at the connection port of said waste solution vessel 28 and the connection tube 27, a communication piece 26 is equipped.

For effecting the priming treatment, the communication piece 25 of the priming vessel 23 and the communication piece 26 of the waste solutoin vessel 28 are broken,and the priming solution 22 in the priming vessel 23 is introduced into the ion concentration adjusting room 6a fromthe inlet 5a through the connection tube 24. According to this operation, the priming solution previously filled up in theion concentration adjusting room 6a is pushed out from theoutlet 9a and recovered in the waste solution vessel 28 through the connection tube 27. Thereafter, the positions nearer to the inlet 5a and the outlet 5b of the connection tubes 24 and 27 are sealed, and each tubes 24 and 27 are cut.

Thus, the priming solution is filled in the ion concentration adjusting room, and the priming treatment device comprising the priming vessel 23 or the waste solution vessel 28, etc., an eluate material eluted from the ion concentration adjusting material can be sterilely and easily washed and removed whereby the blood transfusion device can be preserved for a long period of time.

### Example 7

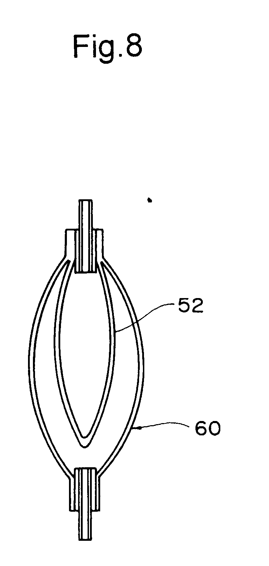

Experimental example A: In the ion concentration adjusting room 6a constituting the blood transfusion device le shown in FIG. 8 was filled 25 ml of a cation exchange resin having a particle size of 0.4 to 1.0 mm as ion adjusting materials 8a, and a physiological saline solution was filled inside of the ion adjusting materials 8a as a priming solution. After sterilizing at 121°C for 20 minutes under high pressure vapor, the blood was preserved at 40°C and a humidity of 75 % for 2 months. Thereafter, breaking communication piece 25 and 26, a priming solution (physiological saline) 22 in a priming vessel 23 was introduced into an ion concentration adjusting room 6a through a connection tube 24, and recovered in a waste solution vessel 28 through an outlet 9a and a connection tube 27 with physiological saline filled in said ian concentration adjusting room 6a, and the position near to an inlet 5a and an outlet 9a of the connection tubes 24 and 27 was sealed. Thereafter, water for injection was passed to the ion concentration adjusting room 6a and a total amount of organic materials was measured. Experimental example B: In order to compare with Experi mental example A, by using the transfusion device 1a (in the ion concentration adjusting room 6a, a cation exchange resin similar to Experimental example A was filled as an ion concentration adjusting material 8a but physiological saline was not filled), it was stored by subjecting to high pressure vapor sterilization treatment in the same manner as in Experimental example A, and thereafter water for injection was passed to the ion concentration adjusting room 6a and a total amount of organic materials was measured. The results are shown in Table 3.

**Table 3**

| | Total organic material concentration (ppm) | | |
|---|---|---|---|
| Sample No. | 1 | 2 | 3 |
| Experimental example B | 63 | 57 | 46 |
| Experimental example A | 0 | 0 | 0 |

From the results shown in Table 3, no organic material was observed after priming and it is confirmed that the eluate material from the cation exchange resin was completely washed and removed.

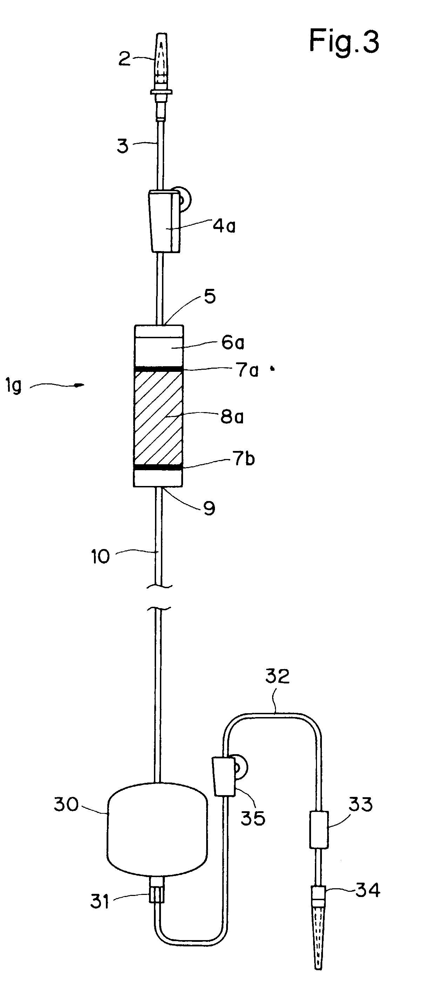

The blood transfusion device 1g shown in FIG. 3 is an example in which a blood storing bag 30 which temporarily store the preserved blood preparation passed through the ion concentration adjusting room 6a is connected to the connection tube 10 which is connected with an outlet 9 of the ion concentration adjusting room 6a of the blood transfusion device shown in FIG. 1. At the outlet of the blood storing bag 30, a blood transfusion tube 32 is connected through a communication piece 31, and to the blood transfusion tube 32, a blood transfusion chamber 33 and a blood transfusion needle 34 are connected. Also, in the course of the blood transfusion tube 32, the roll cramp 35 is provided.

According to the blood transfusion device 1g, the preserved blood preparation passed through the ion concentration adjusting room 6a is temporarily stored in the blood storing bag 30 through the connection tube 10. Next, the communication piece 31 of the blood storing bag 30 is broken to open the blood storing bag 30, and after puncturing the blood transfusion needle 34 to a blood vessel of a patient, adjustment of the blood flow rate is carried out by the roll cramp 35 and the preserved blood preparation in the blood storing bag 30 is transfused to a patent from the blood transfusion needle 34 through the blood transfusion chamber 33.

### Example 8

Each ion concentration adjusting room 6a of the blood transfusion device 1g (Device A) shown in FIG. 3 and the blood transfusion device 1a (Device B) shown in FIG. 1 was filled with 25 ml of a cation exchange resin having a particle size of 0.4 to 1.0 mm. Sample 1 and Sample 2 were each prepared and 800 ml of whole blood was passed through with a flow rate of 50 ml/min, and potassium ion concentrations before and after passing were measured each 100 ml of the filtrated amount (in Device A, measurement was carried out after whole amount was stored in the blood storing bag 30, while in Device B, that flown out from the ion concentration adjusting room 6a was measured as such). The results are shown in Table 4.

**Table 4**

| | Sample 1 K concentration (mEq/l) | | Sample 2 K concentration (mEq/l) | |
|---|---|---|---|---|
| Filtrated amount (ml) | Device B | Device A | Device B | Device A |
| 0 | 30.5 | 30.5 | 59.6 | 59.6 |
| 100 | 0.6 | 5.6 | 7.4 | 13.4 |
| 200 | 1.3 | | 7.6 | |
| 300 | 2.3 | | 9.0 | |
| 400 | 5.1 | | 11.0 | |
| 500 | 7.0 | | 13.9 | |
| 600 | 9.3 | | 17.3 | |
| 700 | 12.4 | | 21.7 | |
| 800 | 13.8 | | 26.3 | |

As clearly seen from Table 4, by providing the blood storing bag 30 at the downstream side and by once storing the preserved blood preparation passed through the ion concentration adjusting room in said bag, particularly when a large amount of the preserved blood preparation is rapidly treated in the ion concentration adjusting room, unevenness of the ion concentrations before and after the treatment can be unified.

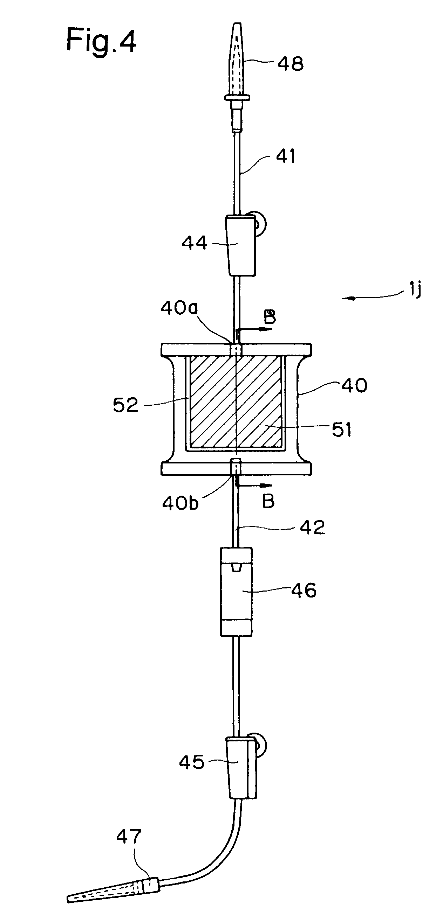

The blood transfusion device 1j of FIG. 4 is a device in which the ion concentrations of the preserved blood preparation are adjusted and simultaneously leukacytes are removed. This blood transfusion device 1j is provided with a blood treatment room 40 and a connection tube 41 is connected at an inlet 40a of the blood treatment room while a blood transfusion tube 42 is connected at an outlet 40b thereof. At the tip portion of the connection tube 41, a connection needle 48 is provided and during the course of the tube 41, a roll cramp 44 is provided. Also, at the bottom portion of the blood transfusion tube 42, an injection needle 47 is provided and during the course of the tube 41, a drip chamber 46 and a roll cramp 45 are provided.

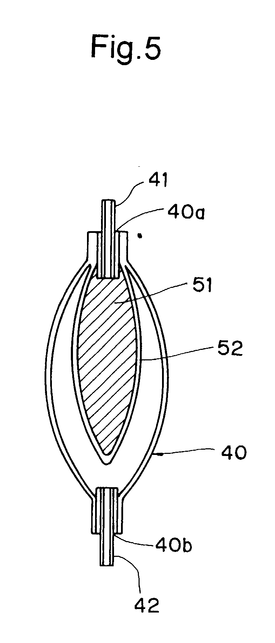

Inside of the blood treatment room 40, ion concentration adjusting materials 51 are provided by being wrapped in a leukocyte-removing filter 52 as shown in FIG. 5. As the ion concentration adjusting materials 51 to be used in this Example, the aforesaid cation exchange resin and anion exchange resin are used and in the leukocyte-removing filter 52, a nonwoven fabric having a diameter of 20µ or less is used.

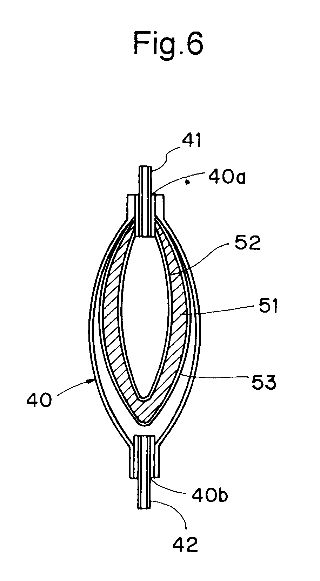

FIG. 6 is a modified example of the blood treatment room 40 wherein ion concentration adjusting materials 51 are packed between a bag-shaped leukocyte-removing filter 52 connected to an inlet 40a and a bag-shaped supporting filter 53 which is broader than said leukocyte-removing filter 52. The supporting filter 53 is constituted by a screen mesh having mesh pores which can retain the ion concentration adjusting materials 51. According to this Example, leukocytes are captured inside the bag-shaped leukocyte-removing filter.

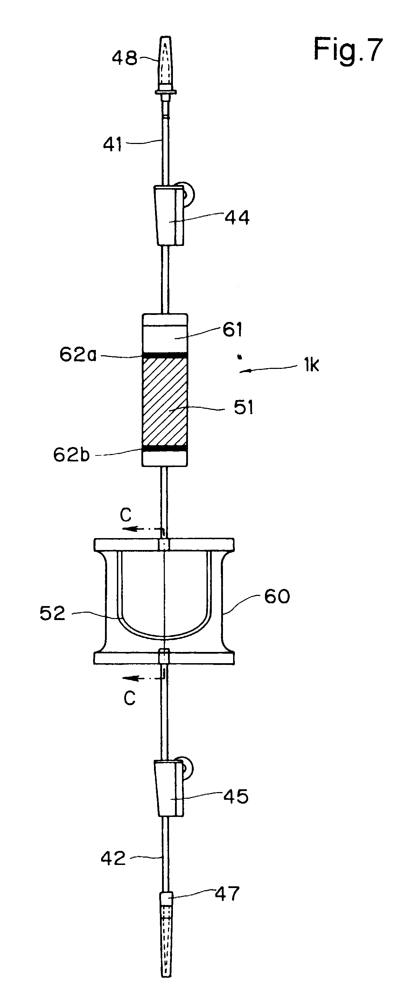

The blood transfusion device 1k of FIG. 7 is an example in which an ion concentration adjusting room 61 and a leukocyte-removing room 60 are separately provided. That is, in the course of the connection tube 41, the ion concentration adjusting room 61 which is the same as in FIG. 1 is provided and the inside of the ion concentration adjusting room 61, the above-mentioned ion concentration adjusting materials 51 are packed between the upper and the lower supporting filters 62a and 62b. Also, a bag-shaped leukocyte-removing filter 52 is provided in the leukocyte-removing room 60 as shown in FIG. 17. According to this solution transporting device 1k, adjustment of an ion concentration and a removing treatment of leukocytes of the preserved blood preparation are separately carried out. The ion concentration adjusting room 61 and the leukocyte-removing room 60 may be provided in the reverse order.

The leukocyte-removing filters shown in FIG. 4 to FIG. 8 may be used in the ion concentration adjusting rooms shown in the above-mentioned FIG. 1 to FIG. 3.

### Example 9

By using the blood transfusion device 1j shown in FIG. 4, the preserved blood preparation was treated according to the following conditions. As a blood preserving solution, 200 ml of blood was preserved in a blood bag (collecting blood volume of 200 ml) made of a soft polyvinyl chloride containing a CDP solution. The preserved blood was introduced into a blood treatment room 40 by adjusting roll cramps 44 and 45 through a connection tube 41 with a head of 70 cm. A flow down speed of the blood was 25 ml/min and the blood flown down from an injection needle 47 was collected in a blood receiving vessel. In a leukocyte-removing filter 52 at the blood treatment room 40 was packed 10 ml of polystyrene sodium sulfonate having an average particle size of 0.4 mm and an exchange ability of 1.8 mEq/ml as ion concentration adjusting materials 51. Potassium ion concentrations in the plasma before and after passing the blood treatment room 40 were measured by using an ion chromatograph device 4500i manufactured by DIONEX Co.

Also, the number of leukocytes before and after passing the leukocyte-removing filter 52 was measured by adding 100µl of the specimen after adding 900µl of a Turk's solution to a test tube, and after stirring the sample with a mixer, allowed to stand to effect hemolysis of erythrocytes for 10 minutes. Total number of the leukocytes of this solution in 40 grids was counted by using a najetchamber with a light microscope. The results 2 are shown in Table 5.

**Table 5**

| Sample No. | 1 | 2 | 3 | 4 |
|---|---|---|---|---|
| K⁺ concentration (mEq/l) Before passing blood treatment room | 9.0 | 13.8 | 23.8 | 33.1 |
| After passing blood treatment room | 3.0 | 4.1 | 8.2 | 9.9 |
| Removed ratio (%) | 66.7 | 70.3 | 65.5 | 70.1 |
| Leukocyte-removing ratio (%) | 99.98 | 99.98 | 99.99 | 99.98 |

As shown in Table 5, the potassium ion concentration in the plasma of the preserved blood preparation can be lowered and simultaneously leukocytes can be removed. Thus, hyperkalemia after blood transfusion can be prevented and side reactions such as GVHD due to leukocyte-removal, etc. can be prevented whereby blood transfusion can be secured.

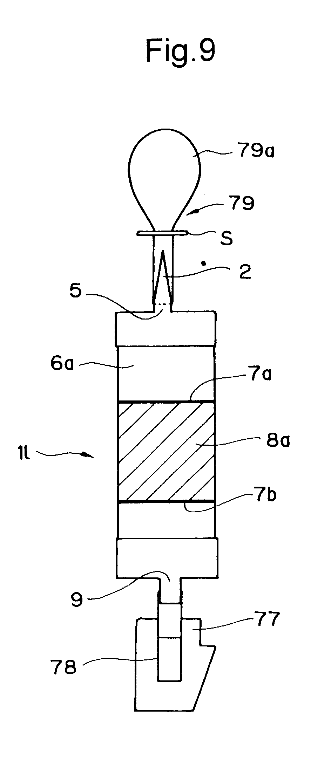

A blood transfusion device 11 shown in FIG. 9 is an example in which the present invention is further progressed and an example wherein a connection tube 3 and a blood transfusion tube 10 are omitted in the blood transfusion device la shown in FIG. 1. In the blood transfusion device 11, a connection needle 2 for introducing a preserved blood is provided at the upper portion of the ion concentration adjusting room 6a and a blood transfusion inlet 78 is provided at the bottom portion of the same, and the blood transfusion inlet 78 is covered with a protector 77 with an airtight structure. The protector 77 is made of a flexible plastic and constituted so as to capable of broken by pulling at a predeter mined portion.

To the connection needle 2, a sealing member 79 is provided. As the sealing member 79, it is preferably having flexibility and a balloon like state which can absorb air expanded in the ion concen tration adjusting room 6a at high pressure vapor sterilization treatment (autoclave sterilization) of the blood transfusion device 11.

In the blood transferring device la shown in FIG. 1, drops of water remain inside of the tubes 3 and 10 after high pressure vapor sterilization treatment so that the tubes 3 and 10 become whiten so that drying for a long period of time is required. However, in the blood transferring device 11, the tubes 3 and 10 are omitted so that such whitening is not occurred whereby drying treatment for a long period of time is not required. Also, various parts such as tubes 3 and 10, roll cramps 4a and 4b in the blood transferring device la can be omitted so that production cost, etc. become cheap, constitution of the whole device is compact and operation becomes simple.

The blood transferring device 11 being assembled as shown in Fig 9 is carried out high pressure vapor sterilization treatment. At this time, air expanded in the ion concentration adjusting room 6a is absorbed by a balloon portion 79a of the sealing member 79 through the connection needle 2. Thereby breakage of the constituting member of the ion adjusting room 6a due to expansion of air and liquid leakage accompanied thereby or the like can be prevented. After carrying out high pressure vapor sterilization treatment, the base portion of the balloon-shaped sealing member 79 is sealed (S in FIG. 9 shows a sealing portion) by high frequency welding, etc. to cut off the balloon portion 79a. The connection needle 2 is covered airtightly with the under portion of the sealing member 79 until use.

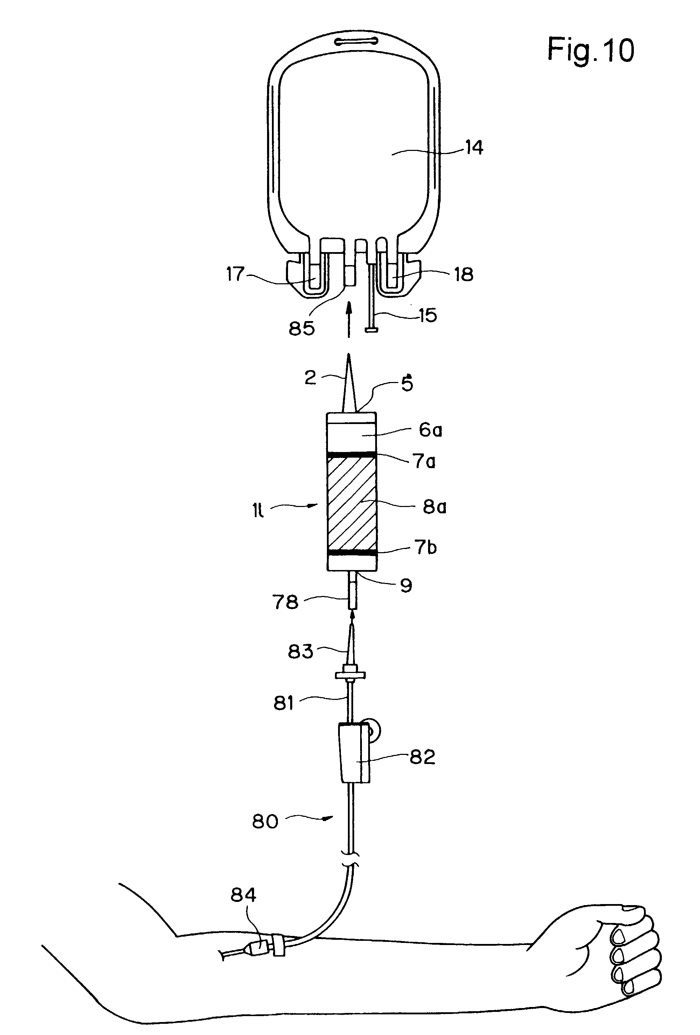

When a preserved blood preparation is transfused to a patient by the blood transfusion device 11, the under portion of the sealing member 79 is taken off from the connection needle 2, a blood transfusion inlet 78 is exposed by breaking a protector 77, and thereafter, a blood transfusion member 80 is connected to the blood transfusion inlet 78 as shown in FIG. 10. The blood trans fusion member 80 is constituted by a blood transfusion tube 81, a blood transfusion needle 83 to be provided at one end portion thereof, an injection needle 84 to be provided at another end portion of the same, and a roll cramp 82 to be provided in the course of the blood transfusion tube 81, and the blood transfusion needle 83 is connected by inserting into the blood transfusion inlet 78 of the blood transferring device 11.

Next, a blood bag 14 in which a preserved blood preparation is contained therein is hanging down at a predetermined height, the connection needle 2 of the liquid transferring device 11 is connected by inserting it into a blood transfusion inlet 85 of the blood bag 14, and the roll cramp 82 which is closing the blood transfusion tube 81 is opened.

Thereby the preserved blood preparation inside of the blood bag 14 is flown out from the injection needle 84 through the connection needle 2, the ion concentration adjusting room 6a and blood transfusion tube 81 in this order due to the head difference. After confirming flown out of the preserved blood preparation from the injection needle 84, blood transfusion is carried out by pricking the injection needle 84 into the blood vessel of a patient and adjusting the flow rate of the preserved blood preparation by the roll cramp 82.

The blood transferring device 11 can be applied also to the blood transferring device 1e shown in FIG. 2. That is, in the same manner as in the blood transferring device 11 shown in FIG. 9, the connection tube 3 or 41 and the blood transfusion tube 10 or 42 are omitted, and in place therefor, the connection needle 2 to which the sealing member 79 is provided at the upper portion of the ion concentration adjusting room 6a is connected, and the blood transfusion inlet 78 covered with-the protector 77 is provided at the bottom portion. The device can be used with the blood transfusion member 80.

When the blood transferring device 11 is applied to the blood transfusion device 1g shown in FIG. 3, the blood storing bag 30 and the blood transfusion chamber 33 are provided in the course of the blood transfusion member 80. That is, the blood transfusion member 80 is constituted, from the upstream side to the downstream side, by the blood transfusion needle 83, the blood storing bag 30, the roll cramp 82, the blood transfusion chamber 33 and the injection needle 84 in this order.

In the case of the blood transfusion device 1j shown in FIG. 4, the drip chamber 46 is provided in the course of the blood transfusion tube 81 of the aforesaid blood transfusion member 80 and the blood transfusion member 80 is constituted, from the upstream side to the downstream side, by the blood transfusion needle 83, the drip chamber 46, the roll cramp 82 and the injection needle 84 in this order.

In the case of the blood transfusion device 1k shown in FIG. 7, the leukocyte-removing room 60 to which the leukocyte-removing filter 52 is provided is provided in the course of the blood transfusion tube 81 of the aforesaid blood transfusion member 80 and the blood transfusion member 80 is constituted, from the upstream side to the downstream side, by the blood transfusion needle 83, the leukocyte-removing room 60, the roll cramp 82 and the injection needle 84 in this order.

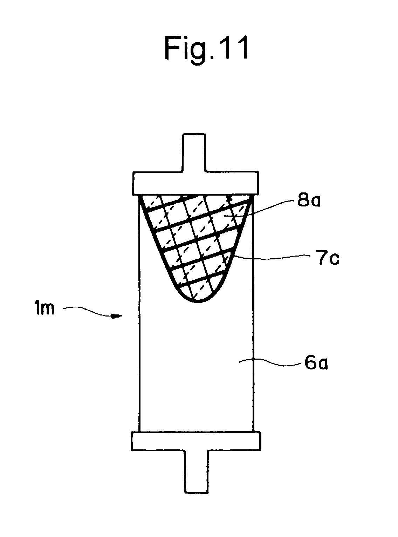

On the other hand, the blood transferring device 1m shown in FIG. 11 is an example in which a hat-shaped filter 7c is provided to the upper end portion at the inside of the ion concentration adjust ing room 6a, and an ion concentration adjusting material 8a is provided inside of the filter 7c. By making the filter 7c to hat-shaped, a filtration surface becomes large as compared to the plate-shaped filters 7a, 7b, 62a and 62b of the above-mentioned ion concentra tion adjusting rooms 6a and 61. Thus, particularly when a blood preparation preserved for a long period of time is filtered, no clogging due to microaggregate (blood aggregate) occurs whereby blood can be rapidly passed therethrough.

The blood transferring device lm can be applied to in place of the ion concentration adjusting rooms 6a and 61 in which plate-shaped filters 7a, 7b, 62a and 62b are provided as shown in the aforesaid blood transfusion device 1a, 1e, 1g, 1i, 1k and 1l.

In the liquid transferring devices 1a to 1m of the present invention as mentioned above, a mixed material of a cation exchange resin and an anion exchange resin can be used as an ion concentration adjusting material 8a or 51. According to the above, both of the potassium ion and the citric acid. ion can be adsorbed simultaneously. Also, an eluate from the cation exchange resin can be adsorbed by the anion exchange resin, and an eluate portion from the anion exchange resin can be adsorbed by the cation exchange resin so that it is possible to depress an amount of the eluate.

### Example 10

Experimental example C: In the ion concentration adjusting room 6a constituting the blood transfusion device 1a shown in FIG. 1 was filled a mixed material of 20 ml of a cation exchange resin (sodium polystyrene sulfonate) having an average particle size of 0.4 mm and 5 ml of an anion exchange resin (trimethyl ammonium hydrochloride) having an average particle size of 0.6 mm as ion adjusting materials 8a, and a physiological saline was filled inside of the ion adjusting materials 8a as a priming solution. After storing at 60 °C for 5 days to 15 days. Thereafter, a total amount of organic materials of the above priming solution was measured.

Experimental example D: In order to compare with Experi mental example C, by using the transfusion device 1a in which 20 ml of a cation exchange resin (sodium polystyrene sulfonate) having an average particle size of 0.4 mm as ion concentration adjusting materials 8a is filled and 25 ml of physiological saline is filled at the inside of the ion concentration adjusting material 8a as a priming solution, a total amount of organic materials was measured in the same manner as in Experimental example C. The results are shown in

**Table 6**

| | Experimental example C | | | Experimental example D | | |
|---|---|---|---|---|---|---|
| Sample No. Stored days | 1 | 2 | 3 | 1 | 2 | 3 |
| 5 days | 25 | 21 | 24 | 65 | 61 | 56 |
| 10 days | 27 | 28 | 26 | 128 | 127 | 121 |
| 15 days | 35 | 37 | 32 | 152 | 155 | 151 |

From the results shown in Table 6, it can be understood that Experimental example C which uses a mixed material of the cation exchange resin and the anion exchange resin shows less detected amount of the organic material than that of Experimental example D which uses the cation exchange resin alone. This is considered that in Experimental example C, an eluate from the cation exchange resin is adsorbed by the anion exchange resin and an eluate from the anion exchange resin is adsorbed by the cation exchange resin.

The effects of the present invention would be clear from the above-mentioned respective Examples. By adjusting the ion concentration in the plasma of the preserved blood preservation, a blood preparation preserved for a relatively long term can be used safety. Thus, washed erythrocytes or fresh blood is not necessarily used and the problems accompanied by the use thereof such as short preservation term, side effects or ensuring preparation can be overcome.

Also, by using an ion concentration adjusting material having excellent ion exchange ability, the ion concentration adjusting material can be minimized, and by increasing uniformity of the particle size thereof, a pressure loss when the preserved blood preparation passes through the ion concentration adjusting material can be suppressed. Therefore, by using a blood pump or a pressure cuff, rapid blood transfusion such as a blood flow rate of 50 ml/min can be realized so that symptoms accompanied by mass blood transfusion including heart operation, etc. can be coped with.

Further, the device of the present invention is as a whole, a closed system including the ion concentration adjusting room and can be sterilized by a high pressure vapor so that there is no fear of migrating bacteria whereby it can be used safety.

## Claims

1. A method of producing a device for transfusing a preserved blood preparation to be infused to a patient, said device comprising an ion concentration adjusting room (6a) which includes:
an inlet (5) for receiving a flow of said preserved blood preparation and an outlet (9) for discharging said flow,
ion exchange material (8a) provided within said ion concentration adjusting room (6a) and arranged to be contacted by said preserved blood preparation when flowing from said inlet (5) to said outlet (9),
said ion exchange material (8a) including a cation exchange resin which has an ion exchange ability of 1 to 5 mEq/ml and an average particle size of 0.2 to 1.5 mmm, and/or an anion exchange resin which has an ion exchange ability of 0.5 to 4 mEq/ml and an average particle size of 0.2 to 1.5 mmm,
the method comprising the steps of
charging said ion concentration adjusting room (6a) with a priming solution harmless to a human body and carrying out a high pressure sterilization procedure on the charged ion concentration adjusting room,
preserving the blood transfusion device during a long term storage period,
thereafter discharging the priming solution from the concentration adjusting room (6a),
and then charging the concentration adjusting room (6a) with a fresh priming solution.

2. The method according to claim 1, wherein the ion exchange material (8a) includes a cation exchange resin adapted to remove ions of at least one of potassium, sodium, and calcium in the preserved blood preparation.

3. The method according to claim 1 or 2, wherein the ion exchange material (8a) includes an anion exchange resin adapted to remove excessive citric acid in the preserved blood preparation.

4. The method according to claim 1 or 2, wherein the ion exchange ability of the cation exchange resin is 1.5 to 3.5 mEq/ml.

5. The method according to claim 1 or 3, wherein the ion exchange ability of the anion exchange resin is 0.8 to 2.5 mEq/ml.

6. The method according to any one of the claims 1 to 5 wherein the average particle size of any said ion exchange resin is 0.2 to 1.5 mm.

7. The method according to any one of the claims 1 to 6, wherein the outlet (9) of the ion concentration adjusting room (6a) is connected to an output tubing (10) leading to an injection needle (11) for injecting the preserved blood preparation to a patient.

8. The method according to any of the claims 1 to 6, further comprising a connection needle (2) for introducing the preserved blood being provided at the inlet portion of the ion concentration adjusting room (6a), and a blood transfusion port (78) covered with a protector (77) being provided at the outlet portion of the ion concentration adjusting room, and a sealing member (79) being provided to the connection needle.

9. The method according to Claim 7, wherein a blood storing portion (30) for temporarily storing the preserved blood preparation passed through said ion concentration adjusting room is provided in the course of the output tubing (10) at the downstream side of the ion concentration adjusting room (6a; 6b).

10. The method according to any one of the Claims 1 to 9, wherein a priming receiving apparatus (23) for introducing the priming solution to the ion concentration adjusting room (6a) and a waste solution apparatus (28) for receiving a waste solution discharged from the ion concentration adjusting room are connected.

11. The method according to any one of the Claims 1 to 10, further comprising a leukocyte-removing filter (52) for removing leukocytes in the preserved blood preparation.

12. The method according to Claim 11, wherein the leukocyte-removing filter (52) is provided in the treating room (40) which contains the ion concentration adjusting materials (51).

13. The method according to Claim 11, wherein the leukocyte-removing filter and the ion concentration adjusting materials are contained in separate rooms (60 and 61), respectively.

## Patentansprüche

1. Verfahren zur Herstellung einer Transfusionsvorrichtung für konserviertes Blutmaterial zur Infusion für einen Patienten, welche eine Ionenkonzentrationseinstellkammer (6a) hat mit
- einem Einlaß (5) zur Aufnahme eines Zuflusses der Blutmaterialkonserve und einen Auslaß (9) zum Austragen des Flusses,
- ein Ionenaustauschmaterial (8a), das in der Ionenkonzentrationseinstellkammer (6a) vorgesehen und so angeordnet ist, daß es von der Blutmaterialkonserve kontaktiert wird, wenn diese vom Einlaß (5) zum Auslaß (9) fließt,
- wobei das Ionenaustauschmaterial (8a) ein erstes Kationenaustauschharz mit einer Ionenaustauschfähigkeit von 1 bis 5 mEq/ml und einer mittleren Partikelgröße von 0,2 bis 1,5 mm und/oder ein Anionenaustauschharz mit einer Ionenaustauschfähigkeit von 0,5 bis 4 mEq/ml und einer mittleren Partikelgröße von 0,2 bis 1,5 mm enthält,
mit folgenden Schritten:
- Beschickung der Ionenkonzentrationseinstellkammer (6a) mit einer für den menschlichen Körper unschädlichen Vorbereitungs(priming)lösung und Durchführung eines Hochdrucksterilisationsprozesses der beschickten Ionenkonzentrationseinstellkammer,
- Aufbewahren der Bluttransfusionsvorrichtung über eine langdauernde Speicherperiode,
- Anschließendes Entleeren der Vorbereitungslösung aus der Konzentrationseinstellkammer (6a), und
- nachfolgendes Beschicken der Konzentrationseinstellkammer (6a) mit einer frischen Vorbereitungslösung.

2. Verfahren nach Anspruch 1, bei welchem das Ionenaustauschmaterial (8a) ein Kationenaustauschharz enthält, das geeignet ist zur Entfernung von Ionen mindestens eines der Bestandteile Kalium, Natrium und Calcium aus der Blutmaterialkonserve.

3. Verfahren nach Anspruch 1 oder 2, bei welchem das Ionenaustauschmaterial (8a) ein Anionenaustauschharz enthält, das geeignet ist zur Entfernung überflüssiger Zitronensäure aus der Blutmaterialkonserve.

4. Verfahren nach Anspruch 1 oder 2, bei dem Ionenaustauschfähigkeit des Kationenaustauschharzes 1,5 bis 3,5 mEq/ml beträgt.

5. Verfahren nach Anspruch 1 oder 3, bei welchem die Ionenaustauschfähigkeit des Anionenaustauschharzes 0,8 bis 2,5 mEq/ml beträgt.

6. Verfahren nach einem der Ansprüche 1 bis 5, bei welchem die mittlere Partikelgröße des Ionenaustauschharzes 0,2 bis 1,5 mm beträgt.

7. Verfahren nach einem der Ansprüche 1 bis 6, bei welchem der Auslaß (9) der Ionenkonzentrationseinstellkammer (6a) an ein Auslaßrohr (10) angeschlossen ist, welches zu einer Injektionsnadel führt zur Injektion der Blutmaterialkonserve bei einem Patienten.

8. Verfahren nach einem der Ansprüche 1 bis 6, wobei am Einlaßbereich der Ionenkonzentrationseinstellkammer (6a) eine Verbindungsnadel (2) zum Einlaß der Blutkonserve vorgesehen ist und ein mit einem Schutz (77) abgedeckter Bluttransfusionsauslaß (78) am Auslaßteil der Ionenkonzentrationseinstellkammer vorgesehen ist und die Verbindungsnadel mit einem Abdichtelement (79) versehen ist.

9. Verfahren nach Anspruch 7, bei welchem ein Blutspeicher (30) zur zeitweiligen Speicherung der durch die Ionenkonzentrationseinstellkammer gelaufenen Blutmaterialkonserve im Verlauf des Auslaßrohres (10) stromabwärts von der Ionenkonzentrationseinstellkammer (6a,6b) vorgesehen ist.

10. Verfahren nach einem der Ansprüche 1 bis 9, bei welchem ein Vorbereitungslösungsgefäß (23) zum Einbringen der Vorbereitungslösung in die Ionenkonzentrationseinstellkammer (6a) und eine Verbrauchtlösungsgefäß (28) zur Aufnahme einer aus der Ionenkonzentrationseinstellkammer entfernten verbrauchten Lösung angeschlossen sind.

11. Verfahren nach einem der Ansprüche 1 bis 10, weiterhin enthaltend ein Leukozyten-Entfernungsfilter (72) zur Entfernung von Leukozyten in der Blutmaterialkonserve.

12. Verfahren nach Anspruch 11, bei welchem das Leukozyten-Entfernungsfilter (52) in dem Behandlungsraum (40) vorgesehen ist, welcher die Ionenkonzentrationseinstellmaterialien (51) enthält.

13. Verfahren nach Anspruch 11, bei welchem das Leukozyten-Entfernungsfilter und die Ionenkonzentrationseinstellmaterialien in getrennten Räumen (60 bzw. 61) enthalten sind.

## Revendications

1. Procédé de production d'un dispositif de transfusion d'une préparation sanguine conservée devant être transfusée à un patient, ce dispositif comprenant une chambre d'ajustement de la concentration en ions (6a) qui comporte un orifice d'entrée (5) pour recevoir un flux de la préparation sanguine conservée et un orifice de sortie (9) pour décharger ce flux, une matière échangeuse d'ions (8a) logée dans la chambre d'ajustement de la concentration en ions (6a) et disposée de manière à être mise en contact avec la préparation sanguine conservée lorsque celle-ci s'écoule de l'orifice d'entrée (5) à l'orifice de sortie (9), la matière échangeuse d'ions (8a) comportant une résine échangeuse de cations qui a une capacité d'échange d'ions de 1 à 5 mEq/ml et une dimension moyenne des particules de 0,2 à 1,5 mm et/ou une résine échangeuse d'anions ayant une capacité d'échange d'ions de 0,5 à 4 mEq/ml et une dimension moyenne des particules de 0,2 à 1,5 mm, caractérisé en ce qu'il comprend les étapes consistant à charger la chambre d'ajustement de la concentration en ions (6a) avec une solution de traitement primaire sans danger pour un corps humain et à effectuer une procédure de stérilisation à haute pression de la chambre d'ajustement de la concentration en ions (6a) chargée, à conserver le dispositif de transfusion sanguine pendant une période de stockage de longue durée, à décharger ensuite la solution du traitement primaire à partir de la chambre d'ajustement de la concentration en ions (6a) et à charger ensuite la chambre d'ajustement de la concentration en ions (6a) avec une solution de traitement primaire neuve.

2. Procédé suivant la revendication 1 caractérisé en ce que la matière échangeuse d'ions (8a) comporte une résine échangeuse de cations adaptée pour extraire des ions d'au moins un élément parmi le potassium, le sodium et le calcium dans la préparation sanguine conservée.

3. Procédé suivant l'une des revendications 1 ou 2 caractérisé en ce que la matière échangeuse d'ions (8a) comporte une résine échangeuse d'anions adaptée pour extraire l'acide citrique en excès dans la préparation sanguine conservée.

4. Procédé suivant l'une des revendications 1 ou 2 caractérisé en ce que la capacité d'échange d'ions de la résine échangeuse de cations est de 1,5 à 3,5 mEq/ml.

5. Procédé suivant l'une des revendications 1 ou 3, caractérisé en ce que la capacité d'échange d'ions de la résine échangeuse d'anions est de 0,8 à 2,5 mEq/ml.

6. Procédé suivant l'une des revendications 1 à 5 caractérisé en ce que la dimension moyenne des particules de la résine échangeuse d'ions est de 0,2 à 1,5 mm.

7. Procédé suivant l'une des revendications 1 à 6 caractérisé en ce que l'orifice de sortie (9) de la chambre d'ajustement de la concentration en ions (6a) est connecté à un tube de sortie (10) aboutissant à une aiguille d'injection (11) servant à injecter la préparation sanguine conservée dans un patient.

8. Procédé suivant l'une des revendications 1 à 6 caractérisé en ce qu'il comprend en outre une aiguille de connexion (2) pour l'introduction de la préparation sanguine conservée, prévue dans la portion d'entrée de la chambre d'ajustement de la concentration en ions (6a), et un orifice de transfusion sanguine (78) recouvert par un capuchon protecteur (77), prévu dans la portion de sortie de la chambre d'ajustement de la concentration en ions (6a), et un organe d'étanchéité (79) prévu sur l'aiguille de connexion.

9. Procédé suivant la revendication 7 caractérisé en ce qu'une portion (30) de stockage du sang, destinée à un stockage temporaire de la préparation sanguine conservée ayant traversé la chambre d'ajustement de la concentration en ions, est prévue sur la longueur du tube de sortie (10) du côté aval de la chambre d'ajustement de la concentration en ions (6a ; 6b).

10. Procédé suivant l'une des revendications 1 à 9 caractérisé en ce qu'un appareil (23) de réception de la solution primaire, destiné à introduire la solution primaire dans la chambre d'ajustement de la concentration en ions (6a), et un appareil (28) de réception de la solution usée, destiné à recevoir une solution usée déchargée à partir de la chambre d'ajustement de la concentration en ions (6a), sont connectés.

11. Procédé suivant l'une des revendications 1 à 10 caractérisé en ce qu'il comprend en outre un filtre (52) d'élimination des leucocytes afin d'extraire des leucocytes de la préparation sanguine conservée.

12. Procédé suivant la revendication 11 caractérisé en ce que le filtre (52) d'élimination des leucocytes est prévu dans la chambre de traitement (40) qui contient les matières (51) d'ajustement de la concentration en ions.

13. Procédé suivant la revendication 11 caractérisé en ce que le filtre d'élimination des leucocytes et les matières d'ajustement de la concentration en ions sont contenus dans des chambres séparées respectives (60,61).
